# NEW EUROPEAN PATENT SPECIFICATION

(11) **EP 0 484 677 B2**
(45) Date of publication and mention of the opposition decision: **05.07.2000**
(45) Mention of the grant of the patent: 12.06.1996
(21) Application number: 91116948.0
(22) Date of filing: 04.10.1991
(51) Int. Cl.: A61B 17/072

(54) **Apparatus for placing staples in laparoscopic or endoscopic procedures**
Vorrichtung zum Anbringen von Klammern bei laparoskopischen oder endoskopischen Eingriffen
Dispositif de pose des agrafes dans les procédures de laparoscopie ou d'endoscopie

(30) Priority: 05.10.1990 US 593654
(43) Date of publication of application: 13.05.1992
(73) Proprietor: United States Surgical Corporation, Norwalk, Connecticut 06856 (US)
(72) Inventor: Green, David T., Westport, Connecticut 06880 (GB); Bolanos, Henry, East Norwalk, CT 06855 (US); Alesi, Daniel E., New Fairfield, CT 06812 (US); Ratcliff, Keith, Sandy Hook, Ct 06482 (US); Sherts, Charles R., Southport, Ct 06490 (US)
(74) Representative: Marsh, Roy David

(56) References cited:
- EP-A- 0 140 552
- EP-A- 0 216 532
- EP-A- 0 369 324
- EP-A- 0 399 699
- EP-A- 0 399 701
- US-A- 4 784 137

## Description

### BACKGROUND OF THE INVENTION

### 1. Field of the Invention

This invention relates to surgical stapling apparatus, and more particularly to a surgical stapler for mechanically effecting a plurality of lines of stitching or ligating staples in body tissue.

### 2. Background of Related Art

In some surgical operations it is necessary to adjoin two hollow body organs alongside each other, generally with their longitudinal axes parallel to each other, and to effect a longitudinal cut through the contacting circumferential walls of the two organs so that the two organs constitute a single hollow chamber along the length of the cut. Correspondingly, the circumferential portions of the two adjoining organs on each lateral side of the cut must be sutured by at least one line of "stitches" in order to maintain the integrity of the union.

Instruments for this purpose can comprise two elongated fingers which are respectively insertable into each organ from an open end thereof, the two fingers thereby having between them the two adjoining walls of the organs. Typically, one of the fingers carries a disposable cartridge housing a plurality of staples arranged in at least two lateral rows while the other finger comprises an anvil for curling the staple legs into hook form upon their being driven against the anvil. The stapling operation is effected by a pusher which travels longitudinally along the cartridge carrying finger extending into one organ, with the pusher acting upon the staples to place rows of staples in body tissue. Immediately behind the pusher and laterally positioned between the staple rows is a knife which severs the facing adjoining walls of the two organs to thereby longitudinally open the two organs to each other between the rows of staples.

One such instrument is disclosed in Bobrov et al. (U.S. Patent No. 3,079,606). The instrument disclosed therein comprises an apparatus for simultaneously making a longitudinal incision and applying a row of staples on both sides of the incision. A further improvement is disclosed in Green (U.S. Patent No. 3,490,675).

A later development disclosed in Green (U.S. Patent No. 3,499,591) applies a double row of staples on each side of the incision. This is accomplished by a cartridge assembly wherein a cam member moves within a guide path between two sets of staggered staple carrying grooves. Staple drive members located within the grooves each have two staple pusher plates, and sloping surfaces disposed within the guide path so as to be contacted by the longitudinally moving cam and be driven along the groove to effect ejection of two staples.

Other instruments use similar structure to mechanically suture and divide organic tubular structures such as, for example blood vessels. With these instruments, the tubular structure to be sutured and/or divided is inserted between the jaws of the cartridge, the cartridge jaws close and a pair of pushers advance and suture the organic structure in two spaced locations with a pair of surgical fasteners. Where dividing is desired, a blade comes forward and divides the tubular structure at a position intermediate the pair of fasteners. Such instruments are shown in U.S. Patent Nos. 3,740,994 and 3,955,581, the disclosures of which are incorporated herein by reference.

These above-mentioned instruments comprise upper and lower frames or replaceable cartridges which must be assembled before use, and disassembled after use. Such instruments have been used successfully in surgical operations requiring the placement of gastrointestinal anastomosis, and ligating and dividing tubular structures but they require the surgeon to have direct manual access to the operation site.

However, in laparoscopic procedures surgery is performed through a small incision, and in endoscopic procedures surgery is performed through narrow endoscopic tubes inserted through small entrance wounds in the skin. Up to now there have been no instruments for placing lateral staple lines in laparoscopic or endoscopic procedures. Nor has there been any instrument suitable for placing lateral lines of staples and cutting tissue therebetween in laparoscopic or endoscopic procedures.

Because endoscopic procedures are more common than laparoscopic procedures, the present invention shall be discussed in terms of endoscopic procedures and apparatus. However, use herein of terms such as "endoscopic", "endoscopically" and "endoscopic portion", among others, should not be construed to limit the present invention to a stapling and cutting apparatus for use only in conjunction with an endoscopic tube. To the contrary, it is believed the present invention may find use in any procedure where access is limited to a small incision, including but not limited to laparoscopic procedures. Also, as used herein the terms "fasteners" and "staples" shall be treated equivalently. Unless otherwise stated, the term "cartridge assembly" shall include at least the cartridge itself and staples or fasteners and staple drive members disposed therein.

Surgical apparatus in accordance with the pre-characterising part of claim 1 below is disclosed in EP-A-0 216 532.

### 3. Objects of the Invention

It is one object of the present invention to provide a surgical stapling apparatus which can adjoin hollow body organs alongside each other, which can be used endoscopically, preferably including cutting apparatus which sutures and divide tubular structure endoscopically, the apparatus having a replaceable cartridge assembly which deactivates after firing, such that any knife is prevented from making multiple cutting passes through the tissue.

### SUMMARY OF THE INVENTION

These and further objects and advantages are achieved by providing a surgical stapling apparatus insertable through a small incision or narrow tube for driving surgical fasteners into body tissue and cutting the body tissue between rows of staples, and as defined in claim 1 below.

In accordance with the present invention a surgical stapling apparatus is provided for placing one or more rows of staples endoscopically. Advantageously, a stapler apparatus constructed in accordance with the invention may further include a knife for making an incision in body tissue between rows of staples. The latter configuration may find particular use in adjoining two hollow organs or in removing an organ, such as the appendix.

In one embodiment of the invention the frame and endoscopic portion of the instrument are reusable with replaceable staple carrying cartridges.

In another embodiment of the invention the endoscopic portion is formed as a disposable unit detachable from a reusable handle portion including the frame.

In yet a further embodiment of the invention, the entire instrument may be constructed as a single-use, disposable unit.

In another embodiment of the invention, the cartridge assembly contains structure for ligating and/or dividing tubular structure.

In use of the present invention, the endoscopic portion of the apparatus is inserted into the body through a small incision or, more likely, through an endoscopic tube. With the anvil member in the open position, body tissue is disposed between the anvil member and the tissue engaging surface of the cartridge assembly. The anvil is then closed against the cartridge to clamp the body tissue between the anvil and cartridge. The instrument is fired so that staples ejected from the cartridge penetrate through the body tissue and are formed closed against the anvil. Where appropriate, a knife forms an incision between several rows of staples. After the instrument has been fired, the clamping action of the anvil and cartridge assembly is released and the endoscopic portion of the instrument is withdrawn from the body.

The present invention advantageously permits a surgeon to perform internal stapling and cutting procedures without full access to the stapling site. Surprisingly, the stapling and cutting instrument in accordance with the invention may be inserted through a small incision or tube in order to place multiple staple lines and make an incision in the stapled tissue between several rows of staples.

The ability to perform stapling and cutting procedures through a small incision or tube remarkably reduces blood loss, tissue trauma and patient recovery time, contributing to improved health care practices.
Fig. 1 to Fig 32B have been cancelled from the specification initially filed because they appear also in Applicant's earlier EP-A-0 399 701. To facilitate comparison between the present patent and the initially filed application the surviving Figures of drawings are not re-numbered.

Reference will now be made, by way of example, to the accompanying drawings, in which:
Fig. 33 illustrates a perspective cutaway view of an assembled stapler apparatus in accordance with a first embodiment of the invention;
Figs. 34 and 34A illustrate an exploded perspective view of the frame and actuating assembly of the stapler apparatus;
Figs. 35 and 35A illustrate an exploded perspective view of elements of the tube assembly of the stapler apparatus in accordance with the second alternative embodiment of the invention;
Figs. 36 and 37 illustrate side and top views of the extension tube;
Figs. 38-40 illustrate a side view in partial cross section, partial top view, and frontal view, respectively, of the rack rod;
Figs. 41-43 illustrate top and side views of the support structure;
Figs. 44, 45 and 45A illustrate a top view, a side view in cross-section and an end view in cross-section of the collar tube;
Figs. 46-49 illustrate top and side cross-sectional views of the upper and lower half of the cover tube;
Figs. 50-53 illustrate side, bottom and cross-sectional views of the channel of the stapler apparatus;
Figs. 54-56 illustrate top, bottom and side views of the anvil;
Figs. 57 and 57A illustrate an exploded perspective and assembled perspective views of the cartridge assembly;
Figs. 58-61 illustrate top, side and frontal views of the cam bar adapter;
Figs. 62-64 illustrate side and top views of the cartridge housing;
Figs. 65-67 illustrate top, bottom and side views of an anvil member in accordance with an alternate embodiment of the present invention for use in ligating tubular tissue;
Figs. 68-69 illustrate an exploded perspective and assembled perspective view of a cartridge assembly in accordance with an alternate embodiment of the present invention for use in ligating tubular tissue;
Figs. 70 and 71 illustrate a side view of clamping jaws in accordance with an alternate embodiment of the jaw members of the present invention; and
Figs. 72 and 73 illustrate a side view of gripping jaws in accordance with an alternate embodiment of the jaw member.

### DETAILED DESCRIPTION OF THE PREFERRED EMBODIMENTS

The surgical apparatus described herein includes a frame and handle portion supporting an endoscopic portion, i.e., an extended tube-like portion having a relatively narrow diameter, on the order of about 10 millimeters, for insertion into a small opening in or tube inserted into the body, such as in the abdominal cavity. The endoscopic portion defines a longitudinal axis and has a length appropriate for reaching the operation site in the interior of the body. The apparatus may be used in conjunction with endoscopes (devices for visually examining the interior of the body, for example, by means of fiber optics). The endoscopic portion of the apparatus is inserted through the small opening or wound, manipulated to the operation site, and the instrument is actuated. The endoscopic portion has a fastening and cutting portion including an elongated housing which carries a fastening and cutting portion including an elongated housing which carries a fastening and cutting portion to the operation site. The fastening and cutting portion includes jaws defined by a staple carrying cartridge (typically located at the distal end of the housing), an anvil and further includes a knife. Actuating the operating portion must be accomplished via intermediate components disposed on or within a narrow longitudinally extending tubular structure. The bulk of the primary operating components are housed on or within a frame, which is located outside the human (or animal) body being operated upon.

The instrument of the present invention has three basic actions or functions.

First, the endoscopic portion is introduced into the body and positioned with the jaws aligned at the stapling site to receive the target tissue. This may involve rotation of the endoscopic portion relative to the body, either by rotating the stapling instrument, as a whole, by rotating the endoscopic portion relative to the frame as permitted in the preferred embodiments, or a combination of both actions.

Second, the instrument has a means for securing the target body tissue between the cartridge assembly and the anvil. This is accomplished by a clamping action. With the target tissue clamped between the anvil and the cartridge assembly, a camming means which surrounds the housing and anvil member is employed to close the jaws of the apparatus and clamp the tissue between the anvil and the tissue contacting surface of the cartridge.

The third action is that of applying the staples to the body tissue. A longitudinally extending channel is employed to deliver longitudinal motion to pusher cam bars and a tissue cutting knife. The cam bars contact pusher elements which drive the staples through the body tissue against the fastener forming or forming surface of the anvil. After the instrument has been fired, the clamping action of the jaws is released and the instrument may be withdrawn from the body. The following details will provide an in-depth understanding of the various elements, operations and functions of the present invention.

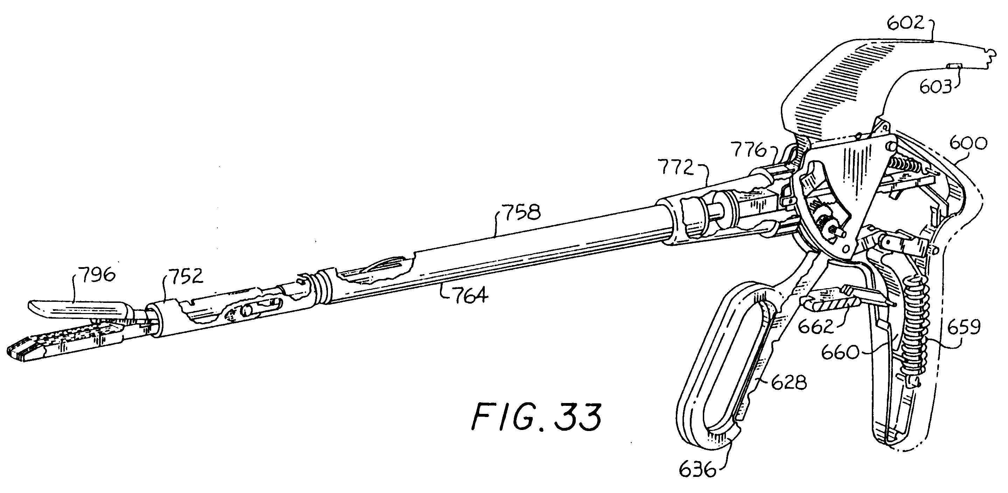

An embodiment of the present invention is illustrated in Figs. 33-64. In the illustrated embodiment the following features are provided. The cartridge assembly deactivates upon firing and is disposable and detachable from the tube assembly, the anvil assembly positively aligns and interfits with the cartridge assembly, the firing handle is provided with a manual safety and the clamp handle and firing handle interlock to prevent accidental firings. Each of these features is described in greater detail below.

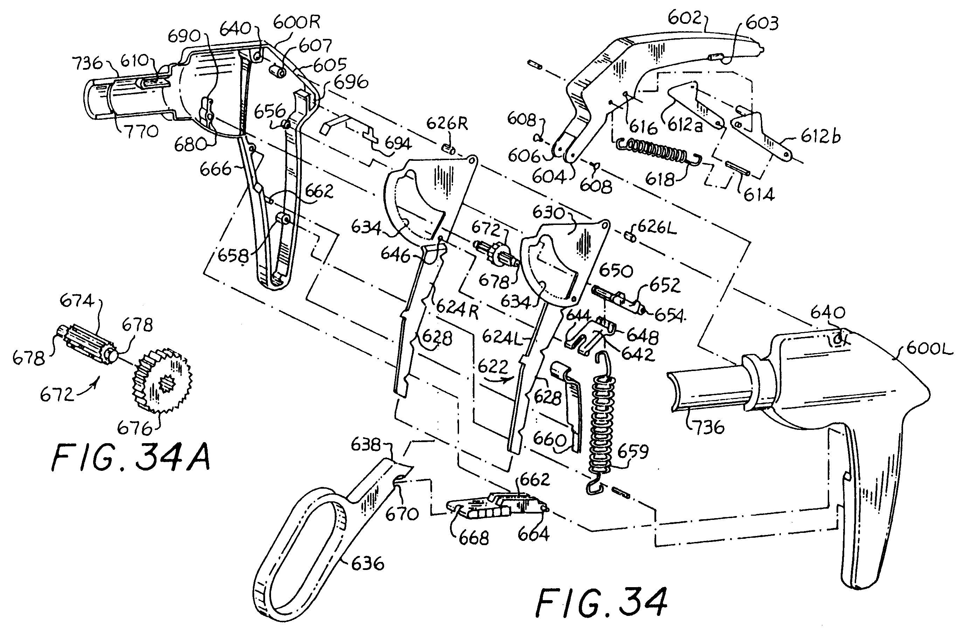

Referring now to Figs. 33, 34 and 34A, frame 600 has two parts, a left portion 600L and a right portion 600R. These portions are optimally fastened together by means of ultrasonic welding along the peripheral contacting surfaces thereof, although screws, adhesives or other means of joining the two body parts also may be used. The frame 600 is of an overall size and shape convenient for being held in the hand.

A clamp handle 602 is pivotally mounted within the frame 600 for articulated movement between an open and a closed position. At a distal end, the clamp handle 602 is provided with a fork portion 604 having a transversely aligned aperture 606 for receiving clamp handle-to-tube pins 608. These pins 608 interfit in slots 610 in the sidewall of the frame 600 for longitudinal reciprocal movement therein. Link pin assembly 612, comprising a pair of links 612a and b interconnected by pin 614, attaches to clamp handle 602 at transverse aperture 616 and to frame 600 wherein pin 614 fits into and is retained by hole 607 in frame 600. A tensioned clamp spring 618 and clamp spring pin 620 connects clamp handle 602 with pin 614 of link pin assembly 612. This spring 618 assists in articulated movement of clamp handle 602 between the closed and open positions. Projections 603 on vertical sides of clamp handle 602 engage the circumferential edge 605 of frame 600 such that when the clamp handle 602 is pivoted down, it is releasably locked into position by the engagement of projections 603 and edge 605.

Gear handle assembly 622 includes left and right braced gear handles, 624L and 624R respectively, and connecting left and right gear handle pins, 626L and 626R. Left and right braced gear handles 624L and 624R are mirror images of each other and have a shank portion 628 and a web portion 630. Aperture 632 is formed in each web portion 630 having, on one internal surface of the aperture, an arcuate rack 634.

When assembled, shank portions 628 of left and right braced gear handles, 624L and 624R, contact in abutting relationship along an internal surface thereof to form a substantially Y-shaped structure with the web portions 630 being separated and substantially parallel. Firing handle 636 is provided with a slot 638 into which shank portions 628 are inserted and held. Transverse apertures 640 are formed in frame portions 600L and 600R to receive gear handle pins 626L and 626R respectively. The gear handle assembly is thus pivotal within frame 600 about transverse apertures 640.

Gear handle assembly 622 further comprises a gear handle link 642 having transverse projections 644 on a distal end thereof. These projections 644 are adapted to pivot about transverse bores 646 in the web 630 of braced gear handles 624 adjacent arcuate racks 634. Gear handle link 642 is further provided with parallel hooked projections 648 on a proximal end which projections are adapted to engage bar 650 on the distal end of a spring link 652. Transverse projections 654 mounted on the proximal end of spring link 652 interfit with holes 656 formed in the frame portions 600L and 600R. A firing handle return spring 659 is connected in tension within frame 600 from projection 658 to spring link bar 650 to provide a mechanical advantage to return the gear handle assembly to its prefiring position after it has been actuated.

A substantially S-shaped kicker spring 660 is mounted within frame 600 and flexes about pin 662. The spring 660 is deflected rearwardly by retracting gear handle assembly 622 and serves to "kick" the assembly back to the original unretracted position.

A manual safety 662 is provided to lock the firing handle 636 in the unfired position to prevent accidental retraction of the gear handle assembly 622. Safety 662 has transverse projections 664 on a proximal end thereof which projections fit into holes 666 in frame portions 600L and 600R to permit pivotal motion of the safety between an engaged position and a disengaged position. In the engaged position, a groove 668, formed in a distal end of safety 662, frictionally interfits with a mating structure 670 on the firing handle 636 to lock the handle in the extended position. To unlock the firing handle 636, the safety 662 is simply pivoted downward out of engagement.

Referring to Fig. 34A, there is shown a pinion spur gear assembly 672 comprising a pinion gear 674 interfitting with a spur gear 676 resulting in plural driving surfaces. Pinion gear 674 is further provided with projections 678 which interfit with holes 680 in frame portions 600L and 600R to facilitate rotational motion of the pinion spur gear assembly 672 about the transverse axis formed by projections 678.

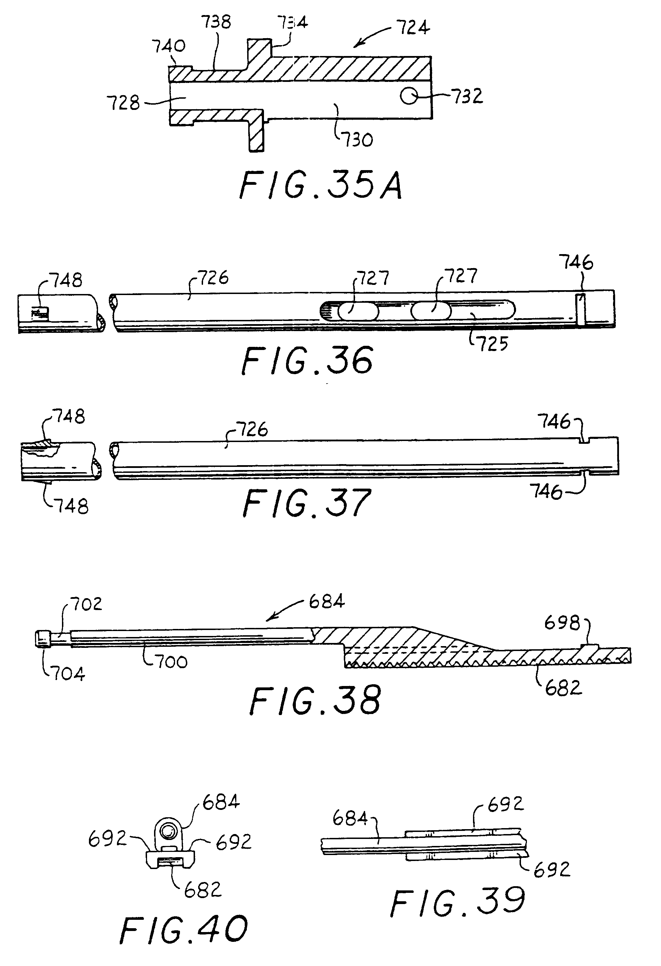

The driving surface formed by the pitch of pinion gear 674 engages arcuate rack 634 such that reciprocal motion of gear handle assembly 622 serves to rotate the pinion spur gear assembly in both clockwise and counterclockwise directions. The driving surface formed by the pitch of spur gear 676 engages a horizontal longitudinal rack 682 formed in the underside of rack rod 684 (Figs. 38-40). Rotation of spur gear 676 translates through the horizontal longitudinal rack 682, to longitudinal reciprocal motion of the rack rod 684.

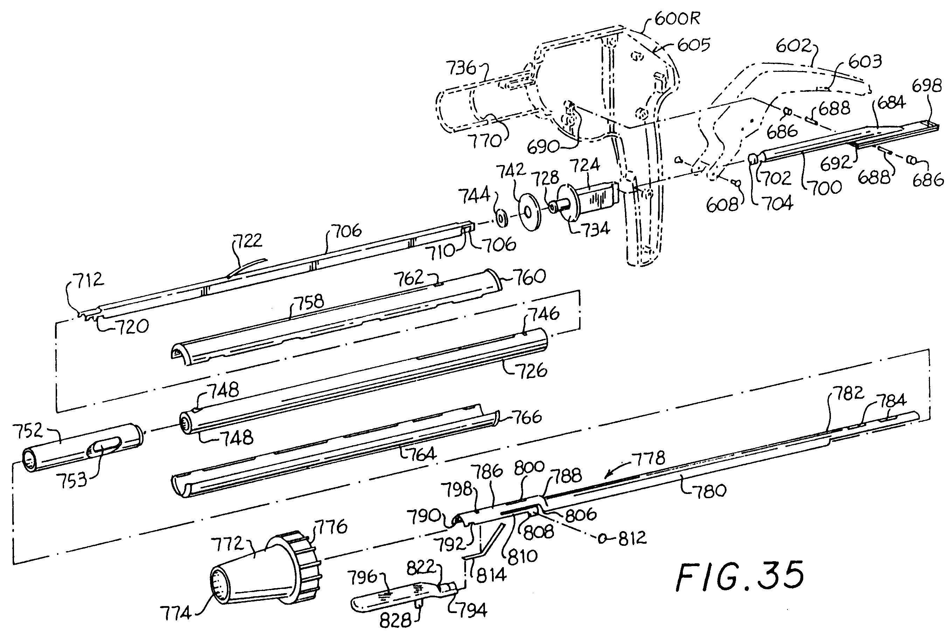

Referring now to Fig. 35 there is shown, in exploded view, the tube assembly 686 of the stapler apparatus in accordance with this second alternative embodiment of the present invention. At a proximal end, rack rod 684 is longitudinally slidable within frame 600 atop spur gear 676. Rollers 686, rotatable fixed in frame portions 600L and 600R by pins 688 engaged in holes 690, abut longitudinal shoulders 692 of the rack rod 682 and serve to prevent the rack rod 682 from disengaging from spur gear 676. Further vertical support for the rack rod is provided by interlock spring 694 (Fig. 34). Interlock spring 694 is formed in a substantially inverted T-shape and mounted transversely in frame 600 with the left and right branches of the "T" fixed into corresponding slots 696 of body portions 600L and 600R. The stalk of the "T" arcs forward to slidably engage a proximal end of horizontal longitudinal rack 682 and maintain a vertical force thereon.

Further, interlock spring 694 serves to assist the unique interlock feature between the gear handle assembly 622 and the clamp handle 602. Referring collectively to Figs. 34 and 35, when clamp handle 602 is extended in the open position, the link pin assembly 612 is pivoted such that a distal end of links 612a and 612b abut the upper proximal surface of rack rod 684 proximate to projection 698. In this position, the link pin assembly 612 abutting projection 698 prevents distal longitudinal motion of the rack rod 684. By providing a vertical force proximate the underside of projection 698, interlock spring 694 prevents the rack rod 684 from pivoting downward to disengage the link pin assembly 612. Similarly, when the clamp handle is closed, link pin assembly 612 pivots about aperture 607 raising the distal ends of links 612a and 612b above projection 698. Thus it is apparent that the gear handle assembly 622 cannot be activated, accidentally or intentionally, when the clamp handle 602 is not closed.

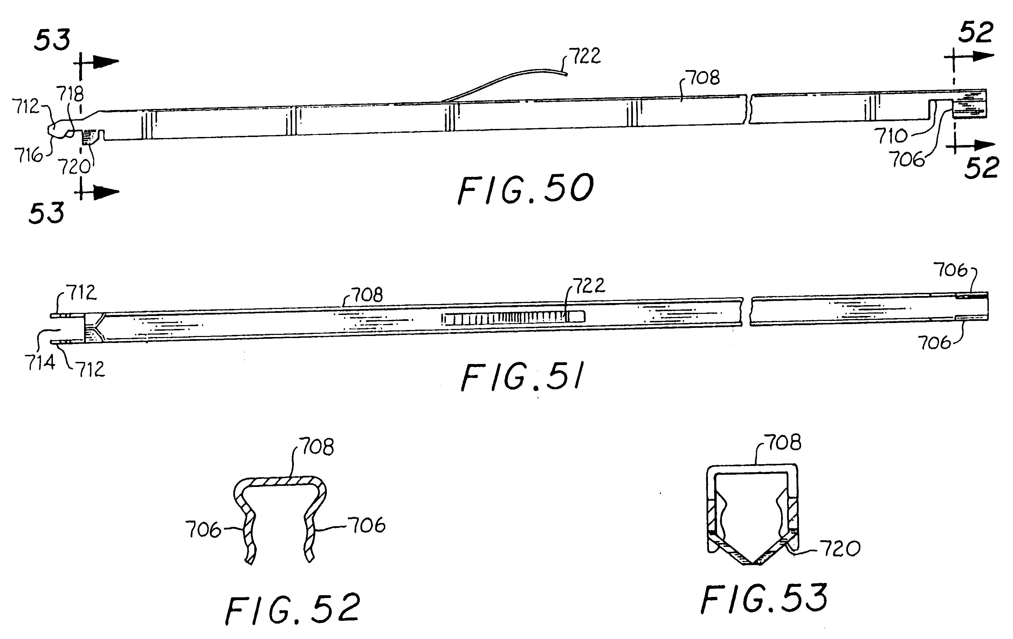

Turning to Figs. 35 and 38, rack rod 684 is provided with a longitudinal cylindrical shaft 700 having an axially mounted protruding stem 702 bearing a push plug 704. Push plug 704 has a chamfered distal tip and is configured and dimensioned to be received between the proximal fingers 706 on channel 708 (Fig. 50-53).

Channel 708 is an elongated piece having a substantially inverted U-shaped cross-section and which is slidably mounted for reciprocal longitudinal motion. As mentioned above, channel 708 has fingers 706 at a proximal end thereof to receive stem 702. Distal to the fingers 706 is a pair of transverse slots 710 dimensioned and configured to receive push plug 704. At a distal end of channel 708 there is provided fork 712 defining a slot 714 therebetween. Fork 712 has a pair of opposed ramping surfaces 716 and 718 respectively, the purposes of which will be described in greater detail below. Proximal to fork 712 is abutting structure 720 which structure extends below the lowermost dimension of fork 712. Biasing spring 722 is positioned on the upper surface of channel 708 and may be integrally stamped from the channel structure. This spring 722 imparts a downward force on the channel 708 to help maintain its horizontal configuration and to assist in engagement and disengagement of the channel 708 with cam bar adapter 846.

A clamp tube 724 is provided to interconnect the clamp handle 602 and an extension tube 726. Referring to Figs. 35 and 35A, clamp tube 724 has a longitudinal bore 728 communicating with a chamber 730. Transverse aperture 732 is formed in a proximal end of the clamp tube 724 and pivotally connects the fork portion 604 of clamping handle 602 by means of pins 608. Push plug 704 of rack rod 684 passes into the proximal end of chamber 730 and out of the distal end of bore 728 such that fingers 706 and slots 710 of channel 708 engage push plug 704 distal to the clamp tube 724.

A flange 734 is formed circumferentially around the periphery of clamp tube 724 and is dimensioned to slidably fit within the area defined by the walls of the tubular portion 736 of frame portions 600L and 600R. Distal to flange 734 of clamp tube 724, a cylindrical section 738 is formed with a locking flange 740 on a distal end thereof. An outer gas seal 742 is positioned around cylindrical section 738 and adhered to the distal vertical face of flange 734. An inner gas seal 744 is positioned distal to outer gas seal 742 in sealing contact with shaft 700 of rack rod 684 and adhered to the distal vertical face of locking flange 740. Both inner and outer gas seals are fabricated of a resilient substantially gas impermeable foam material such as, for example, a closed cell polyethylene foam like Volara, by Voltek. Other materials of construction are encompassed by the present invention and are within the knowledge of one skilled in the art.

Outer seal 742 is maintained in sealing relation with respect to frame 600 by dimensioning the circumference of the outer seal 742 with the inner circumference of the tubular portion 736 of frame 600. In this configuration, clamping tube 724 may move reciprocally longitudinally when driven by the clamp handle 602 while maintaining a substantially sealing relationship between the outer circumference of cylindrical section 738, the inner circumference of outer seal 742, the outer circumference of the outer seal 742 and the inner circumference of the tubular portion 736 of frame 600. Similarly, inner gas seal 744 maintains a substantially sealing relationship between the outer circumference of shaft 700, inner circumference of inner seal 744, the distal vertical surface of locking flange 740 and the proximate vertical surface of inner seal 744.

Referring now to Figs. 35-37 and specifically to Figs. 36 and 37 there is shown extension tube 726 preferably formed as a single tubular structure so as to provide support to the instrument. A pair of transverse slots 746 are formed in a proximal end of extension tube 726 at a point such that when the extension tube 726 is positioned over cylindrical section 738 of clamp tube 724, transverse slots 746 are positioned in the area between the flange 734 and the locking flange 740. In this position, the proximal end of the extension tube up to the transverse slot is crimped effectively locking the extension tube 726 onto the clamp tube 724 while allowing it to freely rotate. An elongated longitudinal aperture 725 is formed in the upper surface of extension tube 726 at a point near the proximal end thereof. A pair of smaller apertures 727 are provided radially apart from aperture 725 to provide access thereto.

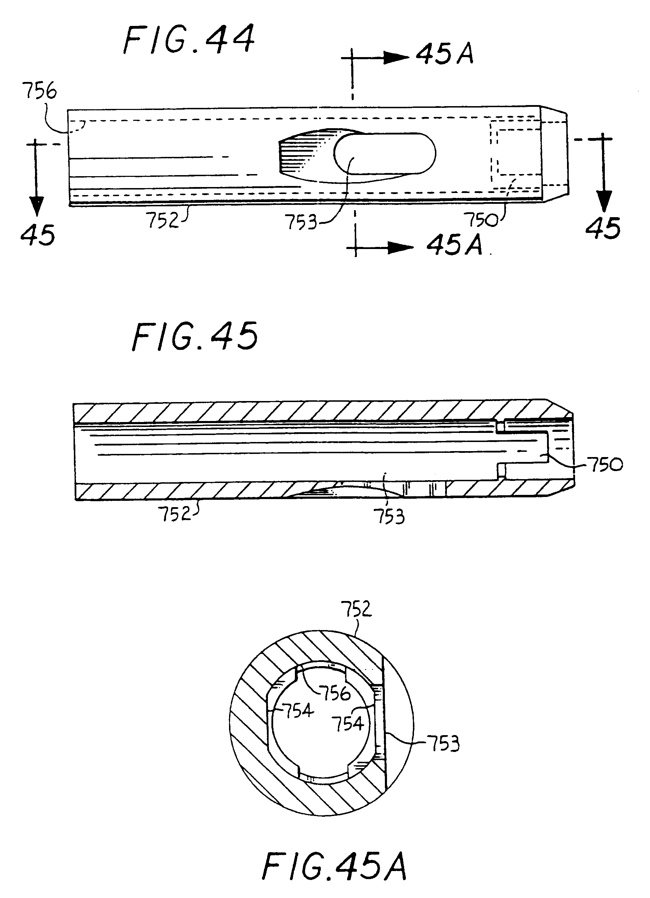

At the distal end of extension tube 726 there is provided a pair of projections 748 which slope outward from the distal end. These projections 748 engage and interlock with slots 750 formed in the internal proximal surface of collar tube 752 (see Figs. 44, 45 and 45A). At its distal end, collar tube 752 is formed with a cross-section having one set of opposed substantially parallel walls 754 and a top arcuate camming surface 756. The arcuate camming surface is described in greater detail below. One side of collar tube 752 is provided with a longitudinal slot 753 for receiving cartridge releasing structure.

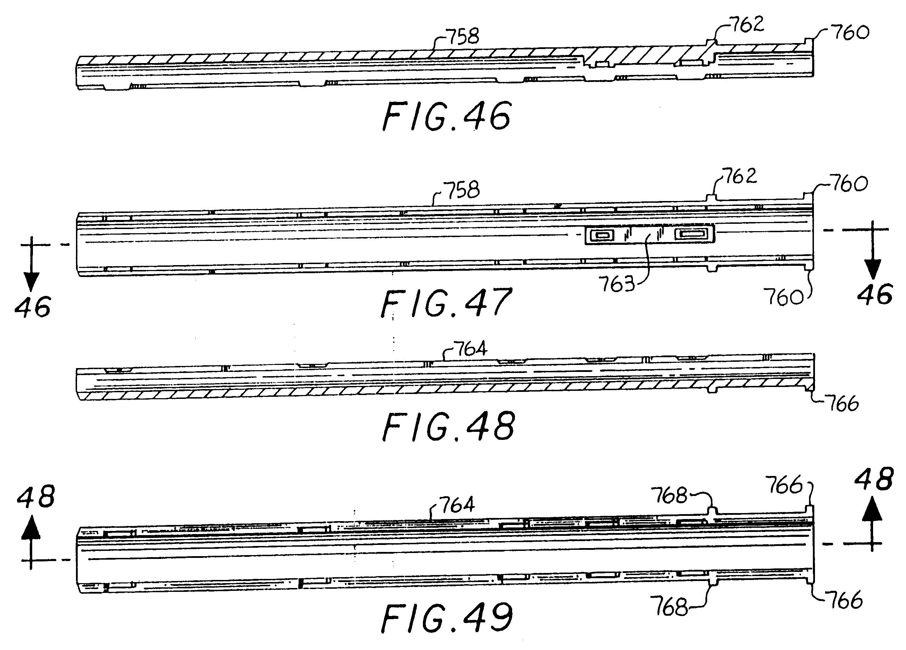

Figs. 46-49 show the upper and lower cover tube structure which surrounds extension tube 726. Upper cover tube half 758 is substantially semi-circular in cross-section having a circumferential flange portion 760 on a proximal end thereof. Set in from the proximal end is a projection 762. Extending from the internal surface is a projecting boss structure 763 which interfits within longitudinal aperture 725 in extension tube 726. This aperture is dimensioned to allow for unrestricted longitudinal motion of extension tube 726.

Lower cover tube half 764 is substantially a mirror image of upper cover tube 758 without the boss structure 763 and includes a flange portion 766 on a proximal end and projection 768 set in from the end. The distal ends of both halfs are chamfered.

Upper and lower cover tube halfs 758 and 764 are joined together, preferably by means of adhesives or ultrasonic welding. Other joining methods are also envisaged and are within the skill of those in the art. When joined, the complete circumferential flange made up of upper and lower portions, 760 and 766 respectively, fit into a circumferential groove 770 formed in the inner surface of the tubular portion 736 of frame 600. Once in position within groove 770, the cover tube is free to rotate about the longitudinal axis relative to the frame 600. The cover tube, however, cannot move longitudinally relative to the body 600.

Referring again to Fig. 35, rotation knob 772 is dimensioned to slide over the cover tube structure as shown and interlock with projections 762 and 768 at a point distal to the tubular portion 736 of frame 600. The rotation knob 772 is in the form of an abbreviated frustoconical structure having a bore 774 therethrough dimensioned to receive the cover tube structure. At a proximal end thereof, knurling 776 may be provided to facilitate rotation. Because of the interlocking structure of the tubes, rotation of the rotation knob 772 effects rotation of the tube assembly.

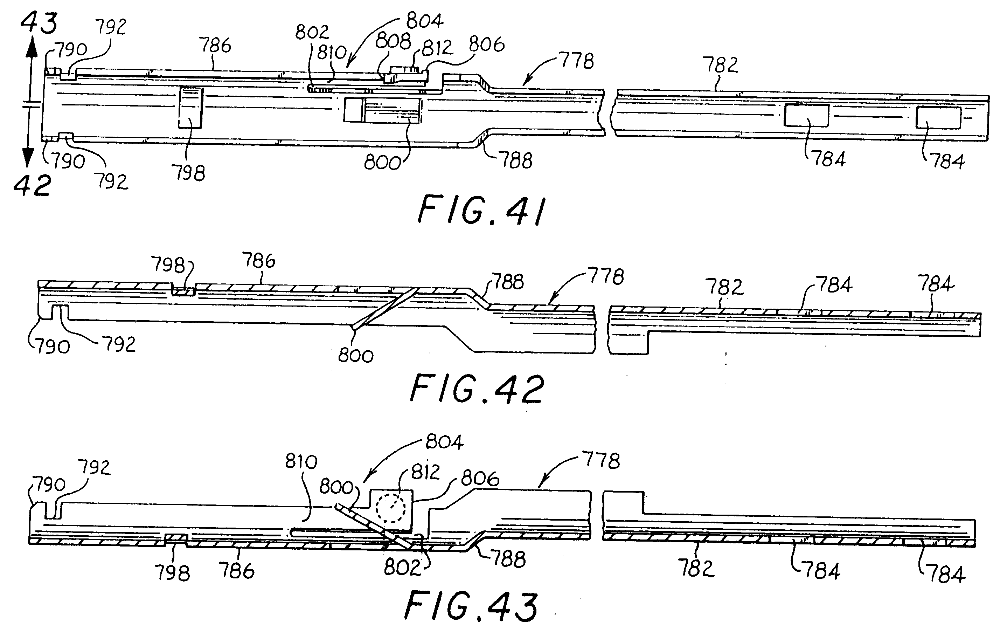

A further element of the tube assembly is support 778 (Figs. 41-43). Support 778 has a central channel portion 780 dimensioned to slidably receive channel 708 for longitudinal reciprocal motion therein. A mounting portion 782 is formed at a proximal end and is provided with attaching slots 784 which are fixed to projecting boss structure 763 of the upper cover tube half 758. Access to the boss structure 763 to effect secure attachment of support 778 is provided through apertures 727 of the extension tube 726. This configuration allows the support 778 to rotate freely with the cover tube while maintaining a fixed longitudinal position with respect to extension tube 726.

At the distal end of support 778 there is provided a mounting and release structure as will be described below. Semi-circular portion 786 is tapered at 788 to attach to channel portion 780. Camming surfaces 790 are formed in the distal end of semi-circular portion 780. A transverse slot 792 is formed proximal to camming surfaces 790 for receiving projections 794 of anvil 796. A transverse crimp 798 is made in the upper surface of semi-circular portion 780 to form a transverse strap for receiving and pivotally holding leaf spring 814 of anvil 796.

Biasing spring 800 is also formed in the upper surface of semi-circular portion 780 and extends inwardly to maintain the channel 708 in the longitudinal horizontal plane as it passes through support 778.

An L-shaped slot 802 is formed in the side wall of semi-circular portion 780 as shown in Fig. 43. This slot 802 forms release catch 804, comprising a rectangular engaging structure 806 having a ramped forward end 808 and a flexible attaching arm 810 which allows engaging structure 806 to be biased inwardly. Release button 812 is positioned on engaging structure 806 and assists in biasing the flexible attaching arm 810 a predetermined distance inward of the semi-circular portion 780. When assembled, semi-circular portion 780 slidably fits within collar tube 752 with release button 812 being accessible through longitudinal slot 753.

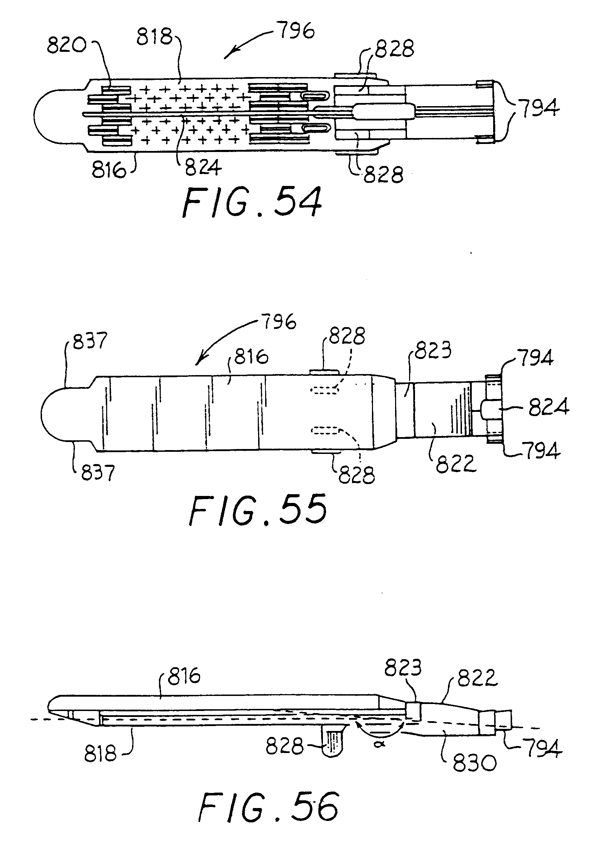

Referring to Figs. 54-56, anvil 796 is an elongated piece which is pivotally mounted in relation to support 778 by means of leaf spring 814. At its distal end, anvil 796 has an anvil plate 816 with a tissue contacting surface 818 having staple forming depressions 820 (See Fig. 54). At its proximal end, anvil 796 is provided with an upper camming surface 822 and locking surface 823 which surfaces are engagable with corresponding top arcuate camming surface 756. Transverse projections 794 are formed at the proximal end of anvil 796 and provide a pivot point about which the anvil 796 may be rotated between an open and closed position by the interaction of camming surface 822, locking surface 823 and top arcuate camming surface 756 of collar tube 752. The radius of curvature of the top arcuate camming surface 756 is shorter than the radius of curvature of camming surface 822 and preferably equal to the radius of curvature of locking surface 823. This configuration prevents flexing of the camming surface 756 of collar tube 752 and lateral movement of the anvil as it is being cammed closed.

Leaf spring 814 is fixed in slot 824 at the proximal end of anvil 796. The angular orientation of the leaf spring 814 is such that, upon insertion of the anvil 796 into the semi-circular portion 786 of support 778, leaf spring 814 passes through the strap formed by transverse crimp 798 and maintains anvil 796 oriented in the open position with projections 794 disposed in transverse slots 792 in the support 778.

Anvil plate 816 also has a longitudinal center groove 824 to permit passage of a knife 826. Anvil 796 provides one of the jaws of the instrument for clamping and securing the body tissue to be fastened. Preferably, anvil 796 is provided with one or more tissue stops 828 to help prevent over-insertion of tissue into the jaws. In a particularly advantageous embodiment shown in Figs. 55-56 the anvil is provided with four tissue stops, two of which are disposed on the outer vertical surface of anvil plate 816 with the remaining two internally transversely positioned. This unique configuration allows for more accurate longitudinal alignment of the jaws and prevents twisting of the anvil upon closure. Anvil 796 is further provided with parallel aligning surfaces 830 positioned below camming surface 822. These aligning surfaces are dimensioned to fit within projections 834 on cartridge housing 832 upon closure of the anvil 796. The engagement of the aligning surfaces 822 and the corresponding projections 834 of cartridge housing 832 serves to more accurately and securely align anvil 796 and cartridge housing 832 upon closure. Further visual confirmation of alignment is facilitated by a pair of parallel longitudinal indentations 837 formed in the distal end of anvil 796. These indentations 837 allow the surgeon to view the closed structure of the anvil 796 and cartridge assembly 836 to confirm accurate longitudinal alignment thereof.

Further, as shown in Fig. 56, the horizontal plane formed by tissue contacting surface 818 intersects the horizontal plane formed by the camming portion of the proximal end of anvil 796 at an obtuse angle "α". This angular orientation pre-cambers the anvil 796 and balances the closure force applied by the anvil 796 to the captured tissue.

As discussed above, a wide variety of staples and fasteners are contemplated for use with the present apparatus. In a preferred embodiment for use with titanium fasteners, it has been found that forming of the fasteners in the staple forming depressions 820 is facilitated by applying a hard, relatively smooth surface on the staple forming portion of the anvil 796. The preferred method of application of this surface is by electroless plating, with the surface being formed of a metallic alloy such as, for example, nickel, gold, silver, titanium nitride or chromium. Where nickel is used, the applied surface is preferably in the range of 100µ - 2000µ in thickness with an optimum thickness of between 200µ - 500µ. Ranges for other alloys may vary depending upon their inherent characteristics.

Where nickel is to be applied, the preferred method is an electroless plating method including the steps of: eletrocleaning the anvil in a cyanide-containing cleaner, reversing polarity at predetermined intervals, preferably about every 10-15 seconds, at a current of about 536 Am⁻² (50 amps/ft²); rinsing thoroughly; rinsing in a solution containing a strong acid, preferably 20% HCL, dipping several times; immersing the anvil in a NiCL strike tank for plating, preferably for two to four minutes at a current of about 536 Am⁻² (50 amps/ft²); rinsing; and immersing the anvil in an electroless Ni bath, preferably Enthone 418 or 431, for a time sufficient to achieve the desired plating thickness. For example, at a deposition rate of 12 µm/hr (.0005 in/hr), a time of between 30 to 40 minutes would be required to achieve a thickness of about 300µ ± 50µ. Other coating procedures are also contemplated including vapor deposition, etc. and are encompassed by the present invention.

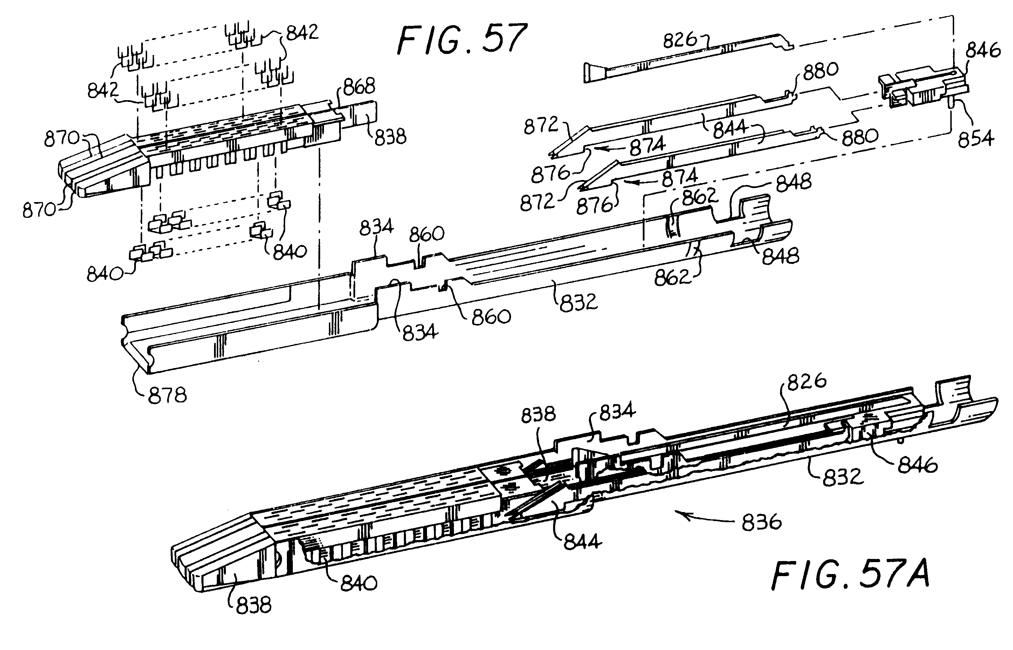

Turning now to Figs. 57-64, there is shown a unique replaceable cartridge assembly 836 in accordance with the present invention. The cartridge assembly 836 includes: a cartridge housing 832; a cartridge 838 having a plurality of pushers 840 and staples 842 disposed in longitudinal arrangement therein; and a plurality of cam bars 844 removably disposed in a cam bar adapter 846 and a knife 826 mounted in the cam bar adapter 846.

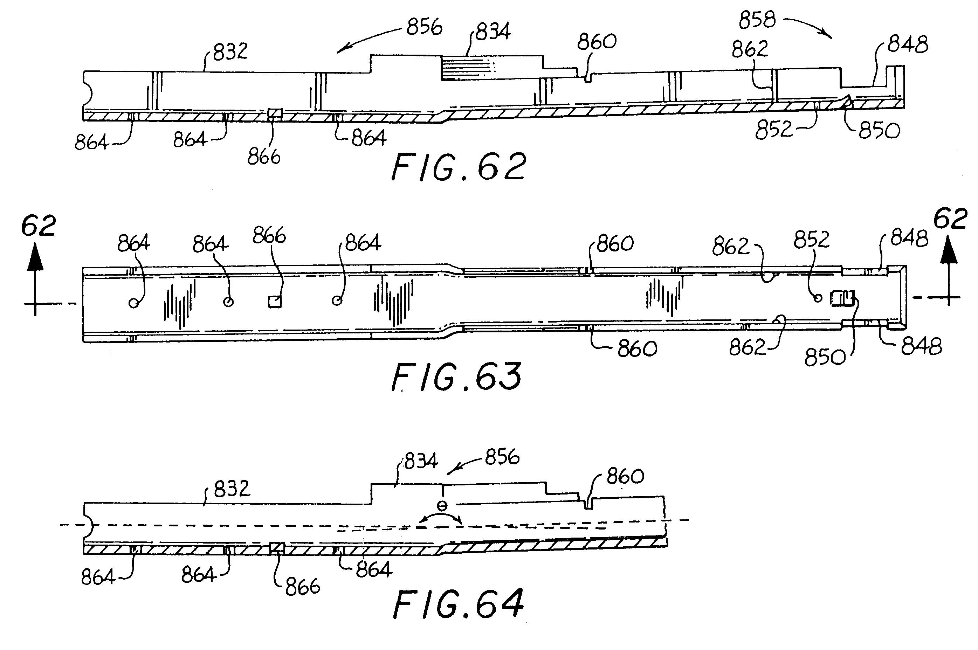

Referring specifically to Figs. 62-64, the proximal end of cartridge housing 832 comprises a substantially elongate channel of semi-circular cross-section having a forward and rearward portion 856 and 858 respectively. A transverse locking slot 848 is formed in rearward portion 858 and serves to engage and retain engaging structure 806 of support 778. Upon insertion into collar tube 752, the ramped forward end 808 of engaging structure 806 is biased inward by the rearward portion 858 of cartridge housing 832 until the engaging structure 806 is totally within and retained by locking slot 848.

Rearward projection 850 is formed in the base of cartridge housing 832. The function of this projection 850 will be described in greater detail below. Forward of the projection 850 is a bore 852 which receives shear pin 854 formed on cam bar adapter 846. A pair of crimps 862 is provided in opposing sidewalls of the rearward portion of the proximal end of the cartridge housing. These crimps 862 provide a friction fit with cam bar adapter 846.

The forward portion 856 of the proximal end of cartridge housing 832 has projections 834 which, upon closure of the cartridge assembly 836 and anvil 796, contact and align on anvil aligning surfaces 830 as described above. A transverse slot 860 is positioned rearward of projections 834 as shown in Figs 62 and 64. This slot serves to receive and retain projections 794 of anvil 796 upon closure of the anvil upon the cartridge assembly 836.

The distal end of the cartridge housing 832 comprises a channel structure of substantially rectangular cross-section. This distal end constitutes the cartridge receiving portion and is dimensioned to receive cartridge 838 therein. Bores 864 and projection 866 serve to engage pins and bores respective in the cartridge 838 so as to align and retain the cartridge 838 within the cartridge receiving portion of the cartridge housing 832.

Referring to Fig. 64, the cartridge receiving portion in the distal end of cartridge housing 832 and the proximal end of cartridge housing 832 are joined at an obtuse angle Θ defined by the intersection of the horizontal planes of both the proximal and distal ends of the cartridge housing 832. This angular orientation serves to pre-camber the cartridge assembly and facilitates accurate closure and alignment of the jaw elements as well as more secure retention of subject tissue.

The cartridge 838 is substantially the same as the cartridge 137 described above and includes longitudinal groove structure 868 for receiving and guiding knife 826 and a plurality of pushers 840 abutting staples 842. The staples 842 are advantageously arranged in six longitudinal rows with three rows positioned on either side of groove structure 868.

Two pairs of longitudinal slots 870 formed in the cartridge housing are adapted to receive a pair of double cam bars 844 therein. Each pair of cam bars serving to drive three corresponding longitudinal rows of staples. Further, the two pairs of longitudinal slots 870 extend to the end of cartridge 838 as shown in Figs. 57 and 57A.

Cam bars 844 are provided with a cam surface 872 in an upper distal end thereof and an overhanging ledge 874 with vertical surface 876 in a lower distal end. This overhanging ledge 874 is dimensioned to extend into the longitudinal slots 870 to a point wherein the vertical surface 876 of the overhanging ledge 874 drops down and abuts the forward edge 878 of the cartridge retaining portion of the cartridge housing when the cam bars 844 move to their distal fired position. At their proximal end, cam bars 844 are provided with hook structure 880 for releasably engaging cam bar adapter 846.

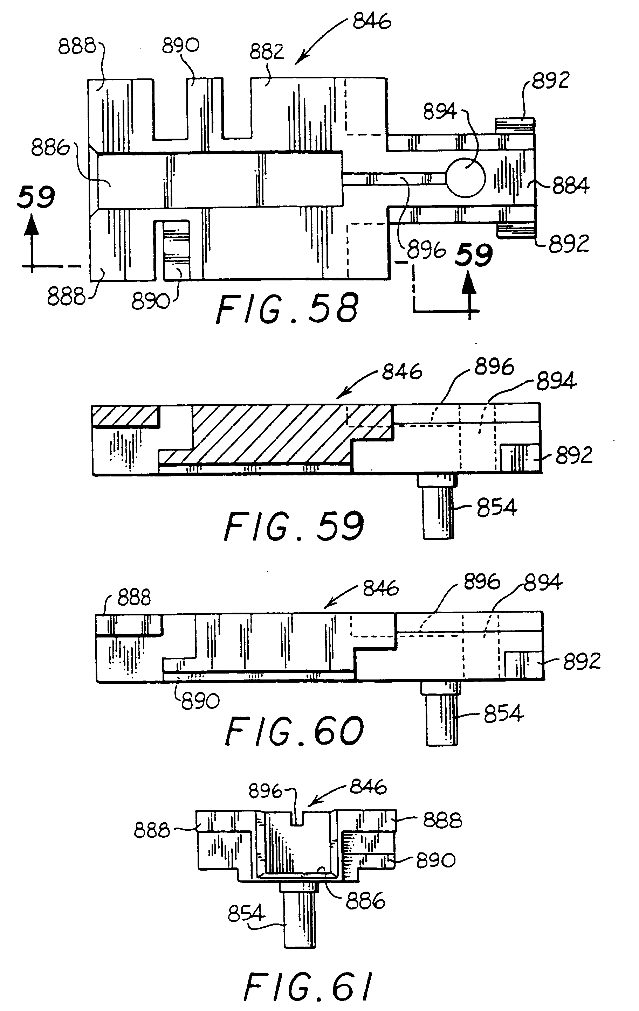

Referring now to Figs. 58-61 there is shown multiple views of the unique cam bar adapter 846 in accordance with one embodiment of the present invention. The cam bar adapter 846 comprises a forward section 882 and a rearward section 884. The forward section 882 is substantially rectangular in shape and has a central longitudinal groove 886 formed therein and dimensioned to receive the longitudinal groove structure 868 therein when the cam bar adapter is urged to its forwardmost position. Flanges 888 and shelves 890 serve to removably retain the proximal end of cam bars 844.

The rearward section 884 is rectangular in shape with projections 892 formed in the proximal end thereof. The rearward section is dimensioned to be receivable within slot 714 of fork 712 in channel 708. The projections 892 are dimensioned to engage ramping surface 716 to allow the fork 712 to ride up and over the projections 892 when the fork 712 is moved in the distal direction.

Vertical bore 894 and longitudinal groove 896 are formed in the rearward section 884 and serve to retain and hold shank 898 of knife 826. Shear pin 854 is integrally formed with cam bar adapter 845 on a bottom surface thereof and, in the prefiring position, is aligned with and receivable into bore 852. Also, in this prefiring position, the rearward section 884 of the cam bar adapter 846 is disposed over rearward projection 850 to effectively shield engagement of abutting structure 720 with projection 850.

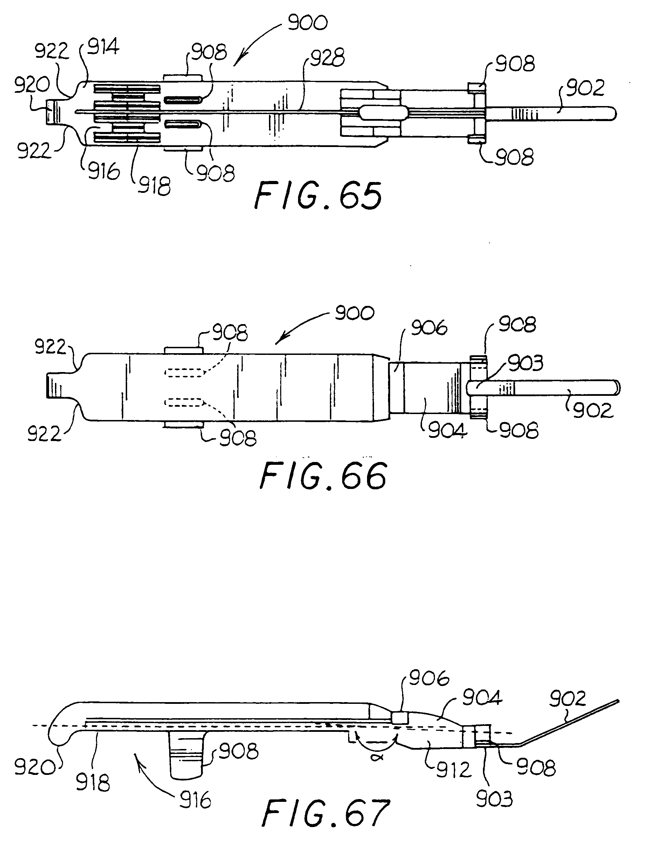

Figs. 65-69 illustrate an embodiment of the cartridge assembly and anvil member of the present invention which permits tubular structures within the body to be ligated and/or divided. Both the cartridge assembly and the anvil member of this embodiment are clamped and actuated by substantially the same frame and tubular structure as that described above with respect to the second alternative embodiment and share substantially the same structure as the cartridge and anvil members shown in other embodiments herein (see Figs. 12-14, 54-56 and 57) with the exception of structure located on the distal ends of both the anvil member and the cartridge assembly.

Referring to Figs. 65-67, anvil 900 is an elongated piece which is pivotally mounted in relation to support 778 by means of leaf spring 902. This leaf spring 902 is fixed in slot 903 at the proximal end of anvil 900 and, when assembled, is retained in the strap formed by transverse crimp 798 in support 778. At its proximal end, anvil 900 is provided with an upper camming surface 904 and a locking surface 906 which surfaces are engagable with corresponding top arcuate camming surface 756 of collar tube 752. Transverse projections 908 are formed at the proximal end of anvil 900 and provide a pivot point about which the anvil 900 may be rotated between an open and closed position by the interaction of camming surface 904, locking surface 906 and top arcuate camming surface 756 of collar tube 752. As in embodiments previously described, the radius of curvature of the top arcuate camming surface 756 of collar tube 752 is shorter than the radius of curvature of camming surface 904 and equal to the radius of curvature of locking surface 906.

Anvil 900 also has tissue stops 908 to prevent overinsertion of tissue into the instrument. Anvil 900 has parallel aligning surfaces 912 positioned below camming surface 904. These aligning surfaces are dimensioned to fit within projections 834 on cartridge housing 832 upon closure of anvil 900.

At its distal end, anvil 900 has an anvil plate 914 with an abbreviated tissue contacting surface 916 having staple forming depressions 918 and an arcuate tissue capturing portion 920. This tissue capturing portion is advantageously designed in a blunted hook configuration as shown in Fig. 67 in order to assist in the capture and proximation of tubular tissue such as, for example, blood vessels, ducts, etc. without unnecessary damage to surrounding tissue. In a particularly preferred embodiment, this arcuate tissue capturing portion is provided with longitudinal tapered surfaces 922. These surfaces fit within mating surfaces 924 in the cartridge assembly 910 and serve to provide the surgeon with a better field of view of the tissue ligation site. Upon closure, surfaces 922 and 924 also give visual confirmation of correct longitudinal alignment. Where captured tissue is to be ligated and divided, a knife 826 is provided and travels in longitudinal groove 928 formed in the anvil plate 914.

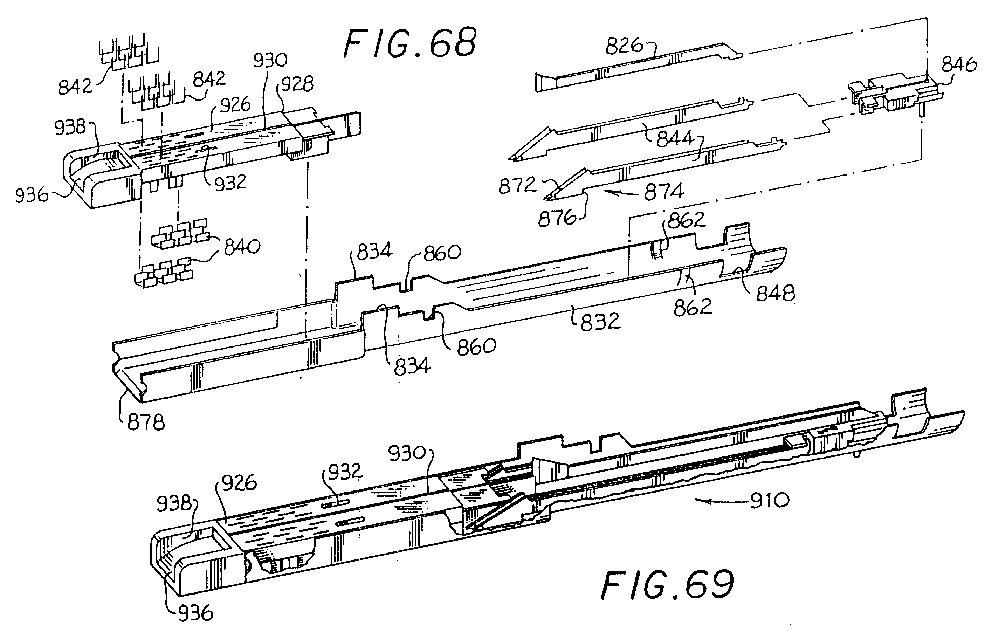

Figs. 68 and 69 illustrate a cartridge assembly 910 in accordance with an embodiment of the present invention for use in grasping, ligating and/or dividing tubular tissue. The cartridge assembly 910 is substantially similar to the other cartridge assemblies described above and includes: a cartridge housing 832; a cartridge 928 having a plurality of pushers 840 and staples 842 disposed in longitudinal arrangement therein; a plurality of cam bars 844 removably disposed in a cam bar adapter 846 and, where dividing is to be effected, a knife 826 mounted to the cam bar adapter 846.

The proximal end of cartridge assembly 910 is substantially the same as that described above with respect to cartridge assembly 836 and is engagable with support 778 in substantially the same manner. Similarly, the distal end of cartridge assembly 910 utilizes the same channel structure of substantially rectangular cross-section.

Cartridge 928 differs somewhat from previous embodiments and includes a plurality of pushers 840 abutting staples 842 arranged in relatively abbreviated longitudinal rows in a tissue receiving surface 926 of cartridge 928. Where a knife 826 is to be used to divide captured, ligated tissue, longitudinal groove structure 930 is provided for receiving and guiding knife 826. In a preferred embodiment, the staples 842 are arranged in six longitudinal rows with three rows positioned on either side of groove structure 930. Bores 932 are provided in the upper surface of cartridge 928 to receive the innermost tissue stops 908.

For typical tubular structure such as blood vessels and ducts, the tissue receiving surface 926 is abbreviated and utilizes fewer staples 842 than previously discussed embodiments. Preferably, the tissue receiving surface 926 is dimensioned to provide sufficient staples to ligate the intended tubular tissue without excess staples being ejected at the site of the tissue ligation.

Two pairs of longitudinal slots 934 formed in cartridge 928 are adapted to receive a pair of double cam bars 844 therein. In this embodiment, each pair of cam bars serves to drive three corresponding longitudinal rows of staples.

The distal end of cartridge 928 has an anvil mating surface 924 formed therein to receive arcuate tissue capturing portion 920 of anvil 900 when the cartridge assembly and anvil are closed. This mating surface comprises an arcuate ramp 936, corresponding in shape to the arcuate tissue capturing portion 920 of the anvil 900, and tapered vertical side walls 938, corresponding to tapered surfaces 922 of anvil 900. These surfaces, 920, 936, 938 and 922 engage upon closure to accurately and positively capture tubular structure for ligating and/or dividing. The operation of cam bars 844 and knife 826 is substantially the same as that described above with respect to Figs. 57-64.

Further alternative embodiments are contemplated in which all or part of the instrument would be disposable. Where the entire instrument constitutes a single use, disposable instrument, the endoscopic portion preferably would be integral with the frame and as much of the instrument as possible would be constructed of plastic. In other contemplated embodiments the cartridge, knife and possibly the anvil might be disposable, alone or as a unit. It is also contemplated, for example, that a replaceable cartridge assembly could be provided which includes the knife and possibly the cam bars.

It is also preferred in all embodiments to include a sealing member within the housing in order to effect an internal seal within the housing. Of course, such a sealing member, not here illustrated, must permit longitudinal movement of the clamping and firing elements.

Suitable materials for use in constructing the instrument in accordance with the invention include stainless steel, titanium, aluminum and plastic. Where disposability of all or part of the instrument is desired, plastic is the material of choice for economic reasons. Plastic is also preferred, where possible, in order to minimize the overall weight of the instrument. Of course, certain parts, such as the anvil, have performance requirements which dictate the material used. In the case of the anvil, the need for high strength and accurately shaped depressions to deform the staples typically requires use of a metal, such as stainless steel. Similarly, the knife requires a fine cutting edge and typically is also made from stainless steel. The staples used with the present invention may be non-absorbable plastic or metal or an absorbable synthetic material, such as a copolymer of polyglycolic acid. Of course, the foregoing identification of materials is exemplary only, and numerous variations, substitutions and changes in material will occur to those of ordinary skill in the art.

### OPERATION OF THE INSTRUMENT

In use, the endoscopic portion of the instrument is inserted into the body, preferably through an endoscopic tube. It is further preferred that the endoscopic tube apparatus be capable of maintaining a sealed pneumoperitoneum, with the internal sealing member of the housing further maintaining this seal despite introduction of the instrument in accordance with the invention into the endoscopic tube. As a practical matter, the jaws of the instrument are closed for insertion into the endoscopic tube, either by pinching the anvil and cartridge prior to insertion or by closing the clamping mechanism prior to insertion.

After insertion into the endoscopic tube, the endoscopic portion may be rotated in order to appropriately orient the instrument at the stapling site. Rotation of the endoscopic portion relative to the body may be attained by rotating the instrument, as a whole, by rotating the endoscopic portion relative to the frame using a finger wheel or a sleeve, or rotation knob 772 (see Fig. 33), or any combination thereof.

Referring to Figs. 33-35, in use, the endoscopic portion of the second alternate embodiment of the invention is inserted into the patient, preferably through an endoscopic tube which can safely and effectively maintain a sealed relationship with the endoscopic portion of the instrument. As in the previously discussed embodiments, the jaws of the instrument are closed for insertion into the endoscopic tube, either by pinching the anvil 796 and cartridge assembly 836 together prior to insertion or by closing the clamping mechanism prior to insertion.

Once inserted into the body cavity, the anvil 796 and cartridge assembly 836 are returned to their first open position (see Fig. 33). By manipulating rotation knob 772 and the instrument, the jaws are oriented to capture the object tissue. Tissue stops 828 in anvil 796 serve to prevent overinsertion of the tissue within the jaws. Once the surgeon is satisfied with the placement of the tissue within the jaws, clamp handle 602 is pivoted downward until it locks in place within frame 600. This pivotal motion forces clamp tube 724, extension tube 726, and collar tube 752 to move longitudinally distal from frame 600. This distal longitudinal movement causes top arcuate camming surface 756 to cam on camming surface 822 forcing anvil 796 to pivot such that: projections 794 move into transverse slot 860; aligning surfaces 830 fit within projections 834; and tissue stops 828 interfit with cartridge assembly 836. Similarly, the pivotal motion of the clamp handle 602 serves to rotate and disengage the link pin assembly 612 from projection 698 on rack rod 684, freeing the rack rod for actuation by the gear handle assembly 622.

When the surgeon is ready to emplace the staples and cut tissue, manual safety 662 is disengaged from firing handle 636 and the firing handle is retracted to proximate the frame 600. This retraction causes arcuate rack 734 to impart counterclockwise rotation on pinion spur gear assembly 672. The counterclockwise motion of the pinion spur gear assembly 672 is translated to distal longitudinal motion by horizontal rack 682. Shaft 700, attached to the proximal end of channel 708 is driven distally causing camming surface 716 of forks 712 to ride up and over projection 892 of the cam bar adapter 845 and drive the cam bar adapter in a distal direction. Shear pin 854 is severed and the cam bars 844 and knife 826 are driven longitudinally through cartridge 838 to sequentially drive and form staples 842 and cut tissue.

At the distal extreme of the longitudinal stroke, the overhanging ledges 874 of cam bars 844 drop over edge 878 of cartridge housing 832 thus abutting vertical surface 876 with edge 878.

After firing, the firing handle 636 is released and returns to its original position with the help of kicker spring 660 and firing handle return spring 659. The return motion of gear handle assembly 622 causes arcuate rack 634 to impart a clockwise rotational motion to the pinion spur gear assembly 672. This clockwise rotational motion is translated to proximal longitudinal motion of shaft 700 by the horizontal rack 682 of rack rod 684. Cam bars 844 are pulled out of cam bar adapter 846 and remain in position in the longitudinal slots 870 of the cartridge 838. The cam bar adapter, with knife 826 attached, moves proximally within cartridge housing 832 until the outer edges of cam bar adapter 846 impinge on crimps 862. At that point, forks 712 of channel 708 are clear of biasing spring 800 in support 778.

The cam bar adapter 846 is held in place by crimps 862 while camming surface 718 of fork 712 causes the fork to ride up and disengage with projection 892 of the cam bar adapter. Channel 708 continues to move in the proximal direction until abutting structure 720 is positioned proximally to rearward projection 850 formed in the floor of cartridge housing 832. At this point, the entire cartridge assembly 836 is deactivated.

In the event that the surgeon should accidentally attempt to again retract the firing handle 636 without replacing the deactivated cartridge with a new unfired cartridge, the resulting distal longitudinal motion of the channel 708 moves abutting structure 720 into contact with rearward projection 850 effectively preventing further movement of forks 712 toward cam bar adapter 846.

After firing, clamp handle 602 is raised with the assistance of clamp spring 618 which action retracts clamp tube 724, extension tube 726 and collar tube 752. This retraction causes leaf spring 814 to move anvil 796 out of engagement of transverse slot 860 and pivot anvil 796 upward. Similarly, raising of clamp handle 602 causes link pin assembly 612 to reengage projection 698 on rack rod 684. In this engaged position, the rack rod 684 is prevented from moving in the distal longitudinal direction in response to an attempted retraction of the gear handle assembly 622.

In order to replace the cartridge assembly, the instrument is withdrawn from the patient. Release button 812 is depressed, biasing engaging structure 806 out of transverse locking slot 848. The cartridge assembly is released and may be removed by pulling it distally out of collar tube 752.

To reinsert a new cartridge assembly, the proximal end of the cartridge assembly is inserted into collar tube 752 until engaging structure 806 locks into transverse locking slot 848. The instrument is now ready for reinsertion and continued use.

Operation of the instrument with the ligating and dividing cartridge and anvil assembly shown in Figs. 65-69 is substantially similar to that described above. Tubular tissue to be ligated and/or divided is captured within the anvil 900 and the cartridge assembly 910 such that the tissue is transversely oriented distal to tissue stops 908 and proximal from arcuate tissue capturing portion 920 of anvil 900. The cartridge assembly 910 and anvil 900 are approximated, effectively interlocking surfaces 920, 936, 938 and 922. The staples 840 are fired, ligating the tissue and, where desired, knife 826 divides the ligated tissue. Opening, removal and replacement of the deactivated cartridge are effected in substantially the same way as described above with respect to the second alternative embodiment.

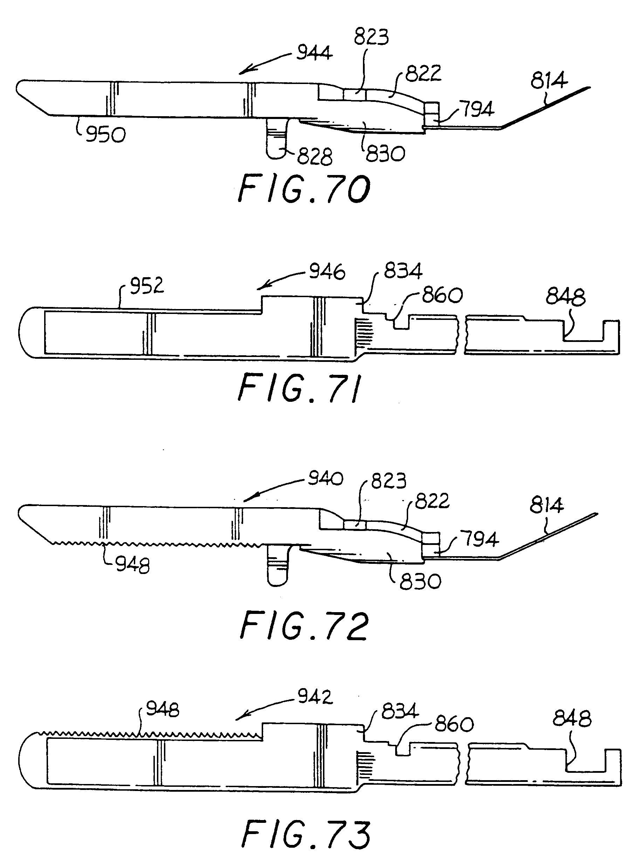

Referring to Figs. 70-73, it is contemplated that the anvil/cartridge assembly of the present invention could be replaced with other interacting jaw members such as, for example, a pair of gripping jaw members, 940 and 942 respectively, for holding and dissecting tissue as well as clamping jaw members, 944 and 946 respectively, for clamping off tissue or portions thereof. These interacting jaw members may include serrated portions 948 to improve gripping/holding ability. Alternatively, the interacting jaw members may be provided with tissue contacting surfaces 950 and 952 respectively, which prevent or minimize trauma to held or clamped tissue.

These interacting jaw members would be mounted in substantially the same way as the anvil/cartridge assembly described herein with the exception that staples and/or knives need not be driven to join and/or divide tissue.

It will be understood that various modifications can be made to the various embodiments of the present invention herein disclosed.

For example, various sizes of the instrument are contemplated, as well as various types of construction materials. Also, various modifications may be made in the configuration of the parts, within the scope of the claims.

## Claims

1. Surgical apparatus for driving surgical fasteners into body tissue comprising:
a) a frame (600);
b) a distal portion (758) defining a longitudinal axis and extending distally from said frame, said distal portion including:
i) an elongated support (778) having a distal member accommodating a replaceable cartridge assembly (836), the replaceable cartridge assembly including a plurality of surgical fasteners (842) slidably mounted therein, and having a tissue engaging surface;
ii) an elongate anvil member (796) having a fastener forming surface (818) and one end pivotally retained in said elongated support such that an anvil member is movable between an open position and a closed position wherein the fastener forming surface is in close co-operative alignment with the tissue engaging surface of the cartridge assembly;
iii) means (602) for moving the anvil member between the open position and the closed position;
iv) means (636) with a stroke for ejecting the surgical fasteners from the cartridge assembly, whereby the fasteners engage the fastener forming surface; and
v) means (850) for disabling the apparatus after a single actuation of the ejecting means (636);
the apparatus being characterised in that:
the anvil member is aligned lengthwise with the longitudinal axis and said one end is the proximal end of the anvil member; in that the apparatus is suitable for endoscopy or laparoscopy and in that the distal portion is an endoscopic portion;
in that:
gas sealing means (742) atmospherically isolate the endoscopic portion and the frame;
and in that:
the means for moving anvil member between the open position and the closed position comprises:
a) on the anvil member (796) a camming surface (822) transversely arcuate with a defined radius of curvature; and
b) a collar tube (752) disposed around at least a portion of the elongated support, said collar tube having a distal camming surface (756) with a defined transverse radius of curvature and further being movable between a first position in which the distal camming surface is located proximally to the proximal end of the arcuate camming surface (822), and a second position in which the distal camming surface is located distally to the proximal end of the arcuate camming surface, the distal camming surface co-operating with the arcuate camming surface such that when the collar tube (752) is moved from the first position to the second position, the arcuate camming surface is urged to the closed position, and wherein the distal camming surface (756) on the collar tube (752) has a radius of curvature which is shorter than the radius of curvature of the arcuate camming surface (822).

2. Apparatus as claimed in claim 1, further comprising means (772) for rotating the endoscopic portion around the longitudinal axis.

3. Apparatus of claim 1 or 2, wherein said means for moving the collar tube between the first position and the second position further comprise:
a) an extension tube (726) extending longitudinally to the frame and having proximal and distal ends;
b) means (602, 724) for longitudinally moving the extension tube relative to the frame; and
c) means (748) for transmitting longitudinal movement from the distal end of the extension tube to the collar tube such that the collar tube is movable between the first position and the second position.

4. Apparatus as claimed in any one of the preceding claims and including a fastener forming surface of titanium nitride or of a metal alloy selected from nickel, gold, silver and chromium.

5. Apparatus as claimed in any one of the preceding claims, wherein the anvil member (796) is pivotally mounted within the elongated support (778) by means of a leaf spring (814).

6. Apparatus as claimed in claim 5, wherein the anvil member is interchangeably mounted within the elongated support.

7. Apparatus as claimed in any one of the preceding claims, further comprising interlock means (612) for preventing actuation when the apparatus is not in a closed position.

8. Apparatus as claimed in any one of the preceding claims, and further comprising manual safety means (662) for blocking retraction of said ejecting means (636).

9. Apparatus as claimed in any one of the preceding claims, wherein the replaceable cartridge assembly comprises:
a cartridge housing (832);
a cartridge holding a plurality of surgical fasteners (842) slidably disposed therein in abutment with corresponding pushers, the cartridge defining a plurality of longitudinal slots (934) accessing the pushers; a cam bar adapter (846) removably mounting a plurality of cam bars (844), the cam bars adapted for longitudinal movement through the longitudinal slots to engage the pushers and eject the staples.

10. Apparatus as claimed in claim 9, further comprising a knife (826), and means for disabling the knife after a single distal pass through the cartridge.

11. Apparatus as claimed in claim 9 or 10, wherein the plurality of cam bars are adapted for a single distal pass through the longitudinal slots, in that the cam bars are detached from the cam bar adapter after a single distal pass through the longitudinal slots and are retained therein to disable the cartridge assembly.

12. Apparatus as claimed in claim 11 as dependent on claim 10 and further wherein the knife is mounted to the cam bar adapter and adapted for longitudinal motion through the cartridge.

13. Apparatus as claimed in claim 12 wherein said means for disabling the knife comprises disengagement means for disengaging a channel (708) of the ejecting means (636) from the cam bar adapter after a single distal pass of the knife through the cartridge.

14. Apparatus as claimed in claim 13, further comprising abutment means for preventing re-engagement of the channel and the cam bar adapter after disengagement thereof.

15. Apparatus as claimed in any one of the preceding claims, and provided with at least one tissue stop (828) to facilitate alignment of the apparatus and prevent over insertion of the tissue therein.

16. Apparatus as claimed in any one of the preceding claims, and arranged with a pistol grip at its proximal end.

## Patentansprüche

1. Chirurgisches Instrument zum Eintreiben von chirurgischen Verbindem in Körpergewebe, umfassend:
a) einen Rahmen (600);
b) einen distalen Teil (758), der eine Längsachse definiert und sieh von dem besagten Rahmen aus in distaler Richtung erstreckt, wobei der besagte distale Teil einschließt:
i) eine langgestreckte Halterung (778) mit einem distalen Element, welches eine austauschbare Magazineinheit (836) beherbergt, wobei die austauschbare Magazineinheit eine Mehrzahl von verschiebbar darin angebrachten chirurgischen Verbindern (842) enthält und eine Gewebeeingriffsfläche aufweist;
ii) ein langgestrecktes Gegenlagerelement (796), das eine Verbinderumformfläche (818) und ein schwenkbar in der besagten langgestreckten Halterung festgehaltenes Ende aufweist, so dass ein Gegenlagerelement zwischen einer offenen Stellung und einer geschlossenen Stellung beweglich ist, wobei die Verbinderumformfläche in enger zusammenwirkender Ausrichtung mit der Gewebeeingriffsfläche der Magazineinheit steht;
iii) eine Einrichtung (602) zum Bewegen des Gegenlagerelements zwischen der offenen Stellung und der geschlossenen Stellung;
iv) eine Ausstoßeinrichtung (636) zum Ausstoßen der chirurgischen Verbinder aus der Magazineinheit, wodurch die Verbinder mit der Verbinderumformfläche in Eingriff treten; und
v) eine Einrichtung (850) zum Inaktivieren des Instruments nach einer einmaligen Betätigung der Ausstoßeinrichtung (636);
wobei das Instrument dadurch gekennzeichnet ist, dass:
das Gegenlagerelement der Länge nach mit der Längsachse ausgerichtet ist und das besagte eine Ende das proximale Ende des Gegenlagerelements ist; dass das Instrument für eine Endoskopie oder Laparoskopie geeignet ist, und dass der distale Teil ein Endoskopteil ist;
und weiter gekennzeichnet durch:
eine Gas-Abdichteinrichtung (742), um den Endoskopteil und den Rahmen atmosphärisch voneinander zu isolieren und dass die Einrichtung zum Bewegen des Endoskopteils zwischen der offenen Stellung und der geschlossenen Stellung umfasst:
a) auf dem Gegenlagerelement (796) eine Nockenoberfläche (822), die in Querrichtung mit einem definierten Krümmungsradius gekrümmt ist; und
b) ein Bundrohr (752), das um mindestens einen Teil der langgestreckten Halterung herum angeordnet ist, wobei das besagte Bundrohr eine distale Nockenoberfläche (756) mit einem definierten Krümmungsradius in Querrichtung aufweist und weiter zwischen einer ersten Stellung, in der die distale Nockenoberfläche proximal vom proximalen Ende der gekrümmten Nockenoberfläche (822) angeordnet ist, und einer zweiten Stellung, in der die distale Nockenoberfläche distal vom proximalen Ende der gekrümmten Nockenoberfläche angeordnet ist, beweglich ist, .wobei die distale Nockenoberfläche derart mit der gekrümmten Nockenoberfläche zusammenwirkt, dass die gekrümmte Nockenoberfläche in die geschlossene Stellung gedrückt wird, wenn das Bundrohr (752) aus der ersten Stellung in die zweite Stellung bewegt wird; und wobei die distale Nockenoberfläche (756) auf dem Bundrohr (752) einen Krümmungsradius aufweist, der kleiner als der Krümmungsradius der gekrümmten Nockenoberfläche (822) ist.

2. Instrument nach Anspruch 1, dadurch gekennzeichnet, dass es weiter eine Einrichtung (772) zum Drehen des Endoskopteils um die Längsachse umfasst.

3. Instrument nach Anspruch 1 oder 2, wobei die besagte Einrichtung zum Bewegen des Bundrohrs zwischen der ersten Stellung und der zweiten Stellung weiter umfasst:
a) ein Verlängerungsrohr (726), das sich in Längsrichtung zum Rahmen erstreckt und ein proximales und distales Ende aufweist;
b) eine Einrichtung (602, 724), um das Verlängerungsrohr in Bezug zum Rahmen in Längsrichtung zu bewegen; und
c) eine Einrichtung (748) zum Übertragen einer Längsbewegung vom distalen Ende des Verlängerungsrohrs auf das Bundrohr, so dass das Bundrohr zwischen der ersten Stellung und der zweiten Stellung bewegbar ist.

4. Instrument nach einem der vorangehenden Ansprüche, dadurch gekennzeichnet, dass es eine Verbinderumformfläche aus Titannitrid oder aus einer unter Nickel, Gold, Silber und Chrom ausgewählten Metall-Legierung enthält.

5. Instrument nach einem der vorangehenden Ansprüche, dadurch gekennzeichnet, dass das Gegenlagerelement (796) mittels einer Blattfeder (814) innerhalb der langgestreckten Halterung (778) schwenkbar angebracht ist.

6. Instrument nach Anspruch 5, dadurch gekennzeichnet, dass das Gegenlagerelement innerhalb der langgestreckten Halterung austauschbar angebracht ist.

7. Instrument nach einem der vorangehenden Ansprüche, dadurch gekennzeichnet, dass es weiter eine Verriegelungseinrichtung (612) umfasst, um eine Betätigung zu verhindern, wenn sich das Instrument nicht in einer geschlossenen Stellung befindet.

8. Instrument nach einem der vorangehenden Ansprüche, dadurch gekennzeichnet, dass es weiter eine manuelle Sicherungseinrichtung (662) umfasst, um ein Zurückziehen der besagten Ausstoßeinrichtung (636) zu blockieren.

9. Instrument nach einem der vorangehenden Ansprüche, dadurch gekennzeichnet, dass die austauschbare Magazineinheit umfasst:
ein Magazingehäuse (832);
ein Magazin, das eine Mehrzahl von verschiebbar darin angeordneten chirurgischen Verbindern (852) im Anschlag mit entsprechenden Schiebern enthält, wobei das Magazin eine Mehrzahl von Längsschlitzen (934) begrenzt, die einen Zugang zu den Schiebern bilden; einen Nockenstangenadapter (846), der eine Mehrzahl von Nockenstangen (844) entnehmbar hält, wobei die Nockenstangen für eine Längsbewegung durch die Längsschlitze angepasst sind, um mit den Schiebern in Eingriff zu treten und die Klammern auszustoßen.

10. Instrument nach Anspruch 9, dadurch gekennzeichnet, dass es weiter ein Messer (826) umfasst, sowie eine Einrichtung, um das Messer nach einem einzigen distalen Hindurchtritt durch das Magazin zu inaktivieren.

11. Instrument nach Anspruch 9 oder 10, dadurch gekennzeichnet, dass die Mehrzahl von Nockenstangen für einen einzigen distalen Hindurchtritt durch die Längsschlitze geeignet sind, dass die Nockenstangen nach einem einzigen distalen Hindurchtritt durch die Längsschlitze vom Nockenstangenadapter gelöst und darin festgehalten werden, um die Magazineinheit zu inaktivieren.

12. Instrument nach Anspruch 11, rückbezogen auf Anspruch 10, dadurch gekennzeichnet, dass weiter das Messer am Nockenstangenadapter angebracht und für eine Längsbewegung durch das Magazin geeignet ist.

13. Instrument nach Anspruch 12, dadurch gekennzeichnet, dass die Einrichtung zum Inaktivieren des Messers eine Ausrückeinrichtung zum Ausrücken einer Rinne (708) der Ausstoßeinrichtung (636) aus dem Nockenstangenadapter nach einem einzigen distalen Hindurchtritt des Messers durch das Magazin umfasst.

14. Instrument nach Anspruch 13, weiter umfassend eine Anschlageinrichtung, um einen erneuten Eingriff der Rinne und des Nockenstangenadapters nach ihrem Ausrücken zu verhindern.

15. Instrument nach einem der vorangehenden Ansprüche, dadurch gekennzeichnet, dass es mit mindestens einem Gewebeanschlag (828) versehen ist, um ein Ausrichten des Instruments zu erleichtern und ein zu weites Einführen des Gewebes in dieses zu verhindern.

16. Instrument nach einem der vorangehenden Ansprüche, dadurch gekennzeichnet, dass es mit einem Pistolengriff an seinem proximalen Ende ausgestattet ist.

## Revendications

1. Appareil chirugical pour chasser des attaches chirurgicales dans du tissu corporel comprenant :
a) un châssis (600) ;
b) une portion distale (758) définissant un axe longitudinal et s'étendant distalement depuis ledit châssis, ladite portion distale incluant;
i) un support oblong (778) comportant un élément distal logeant un ensemble de cartouche échangeable (836), l'ensemble de cartouche échangeable incluant une pluralité d'attaches chirurgicales (842) installées de façon coulissante dans celui-ci et comportant une surface de mise an prise avec le tissu;
ii) un élément d'enclume oblong (796) comportant une surface tonnant les attaches (818) et une extrémité retenue de façon pivotante dans ledit support oblong, de façon qu'un élément d'enclume soit déplaçable entre une position ouverte et une position fermée, où la surface formant les attaches est an un alignement de coopération étroite avec la surface de mise an prise avec le tissu de l'ensemble de cartouche;
iii) un moyen (602) pour déplacer l'élément d'enclume entre la position ouverte et la position fermée;
iv) un moyen (636) avec une course pour éjecter les attaches chirurgicales de l'ensemble de cartouche, par quoi les attaches sont mises an prise avec la surface formant les attaches; et
v) un moyen (850) pour mettre hors service l'appareil après un seul actionnement du moyen d'éjection (636);
l'appareil étant caractérisé an ce que:
l'élément d'enclume est aligné dans le sens de la longueur avec l'axe longitudinal, et ladite extrémité précitée est l'extrémité proximale de l'élément d'enclume; en ce que l'appareil COnvient pour l'endoscopie ou la laparoscopie et en ce que la portion distale est une portion endoscopique;
en ce que:
un moyen étanche aux gaz (742) pour isoler atmosphériquement la portion endoscopique et le châssis;
et en ce que:
le moyen pour déplacer l'élément d'enclume entre la position ouverte et la position fermée comprend:
a) sur l'élément d'enclume (796) une surface de came (822) transversalement arquée avec un rayon de courbure défini; et
b) un tube de collier (752) disposé autour d'au moins une portion du Support oblong, ledit tube de collier ayant une surface de came distale (756) avec un rayon de courbure transversal défini et étant déplaçable en outre entre une première position dans laquelle la surface de came distale est située proximalement à l'extrémité proximale de la surface de came arquée (822), et une deuxième position dans laquelle la surface de came distale est située distalement à l'extrémité proximale de la surface de came arquée, la surface de came distale coopérant avec la surface de came arquée de façon que lorsque le tube de collier (752) est déplacé de la première position à la deuxième position, la, surface de came arquée est sollicitée vers la position fermée, et où la surface de came distale (756) sur le tube de collier (752) a un rayon de courbure qui est plus court que le rayon de courbure de la surface de came arquée (822).

2. Appareil selon la revendication 1, comprenant en outre un moyen (772) pour faire tourner la portion endoscopique autour de l'axe longitudinal.

3. Appareil selon la revendication 1 ou 2, où lesdits moyens pour déplacer le tube de collier entre la première position et la deuxième position comprennent en outre :
a) un tube d'extension (726) s'étendant longitudinalement au châssis et comportant des extrémités proximale et distale;
b) un moyen (602, 724) pour déplacer longitudinalement le tube d'extension relativement au châssis; et
c) un moyen (748) pour transmettre un mouvement longitudinal de l'extrémité distale du tube d'extension au tube de collier de façon que le tube de collier soit déplaçable entre la première position et la deuxième position.

4. Appareil selon l'une des revendications précédentes et incluant une surface formant les attaches en nitrure de titane ou en un alliage métallique choisi parmi le nickel, l'or, l'argent et le chrome.

5. Appareil selon l'une des revendications précédentes, dans lequel l'élément d'enclume (796) est installé de façon pivotante dans le support oblong (778) au moyen d'un ressort à lames (814).

6. Appareil selon la revendication 5, dans lequel l'élément d'enclume est monté de façon interchangeable dans le support oblong.

7. Appareil selon l'une des revendications précédentes, comprenant en outre un moyen d'interverrouillage (612) pour empêcher un actionnement lorsque l'appareil n'est pas dans une position fermée.

8. Appareil selon l'une des revendications précédentes, et comprenant en outre un moyen de sureté manuel (662) pour bloquer le retrait dudit moyen d'éjection (636).

9. Appareil selon l'une des revendications précédentes, dans lequel l'ensemble de cartouche échangeable comprend:
un boîtier de cartouche (832);
une cartouche tenant une pluralité d'attaches chirurgicales (842) disposées de façon coulissante dans celle-ci en butée avec des poussoirs correspondants, la cartouche définissant plusieurs fentes longitudinales (934) permettant l'accès des poussoirs; un adaptateur (846) des barres de came sur lequel sont montées amoviblement une pluralité de barres de came (844), les barres de came étant conçues pour un mouvement longitudinal à travers les fentes longitudinales pour une mise en prise avec les poussoirs et pour l'éjection des agrafes.

10. Appareil selon la revendication 9, comprenant en outre un couteau (826), et un moyen pour désactiver le couteau après une seule passe distale à travers la cartouche.

11. Appareil selon l'une des revendications 9 ou 10, dans lequel la pluralité des barres de came est conçue pour une seule passe distale à travers les fentes longitudinales, en ce que les barres de came sont détachées de l'adaptateur des barres de came après une seule passe distale à travers les fentes longitudinales et sont retenues dans celles-ci pour désactiver l'ensemble de cartouche.

12. Appareil selon la revendication 11 tel que dépendant de la revendication 10, et dans lequel le couteau est monté en outre sur l'adaptateur des barres de came et est conçu pour un mouvement longitudinal à travers la cartouche.

13. Appareil selon la revendication 12, dans lequel ledit moyen pour désactiver le couteau comprend un moyen de dégagement pour mettre hors prise un canal (708) du moyen d'éjection (636) de l'adaptateur des barres de came après une seule passe distale du couteau à travers la cartouche.

14. Appareil selon la revendication 13, comprenant en outre un moyen de butée pour empêcher une remise en prise du canal et de l'adaptateur des barres de came après la mise hors prise de ceux-ci.

15. Appareil selon l'une des revendications précédentes, et équipé d'au moins un arrêt de tissu (828) pour faciliter l'alignement de l'appareil et pour empêcher une insertion excessive du tissu dans celui-ci.

16. Appareil selon l'une des revendications précédentes, et équipé d'une poignée pistolet à son extrémité proximale.
